# EUROPÄISCHE PATENTSCHRIFT

(11) **EP 1 987 859 B1**
(45) Veröffentlichungstag und Bekanntmachung des Hinweises auf die Patenterteilung: **08.06.2011**
(21) Anmeldenummer: 08103398.7
(22) Anmeldetag: 07.04.2008
(51) Int. Cl.: A61N 5/10, H05H 13/00

(54) **Partikeltherapieanlage**
Particle therapy device
Installation de thérapie à particules

(30) Priorität: 02.05.2007 DE 102007020599
(43) Veröffentlichungstag der Anmeldung: 05.11.2008
(73) Patentinhaber: SIEMENS AKTIENGESELLSCHAFT, 80333 München (DE)
(72) Erfinder: Hansmann, Thomas, 69117, Heidelberg (DE); Rietzel, Eike, 64289, Darmstadt (DE)

(56) Entgegenhaltungen:
- EP-A- 1 430 932
- EP-A- 1 477 206
- DE-A1- 19 907 098
- MIZOTA M ET AL: "THE HIGH-ENERGY BEAM-TRANSPORT SYSTEM FOR HIMAC" MITSUBISHI ELECTRIC ADVANCE, MITSUBISHI ELECTRIC CORPORATION, TOKYO, JP, Bd. 62, 1. Januar 1995 (1995-01-01), Seiten 2-04, XP000905407 ISSN: 0386-5096

## Beschreibung

Die Erfindung betrifft eine Partikeltherapieanlage mit zumindest zwei Beschleunigungseinheiten, mit denen jeweils insbesondere eine von zwei unterschiedlichen Partikelarten beschleunigt werden kann. Derartige Partikeltherapieanlagen finden Einsatz insbesondere bei der Behandlung von Tumoren.

Die Partikeltherapie ist ein Verfahren der Strahlentherapie, bei dem ein zu behandelndes Gewebe mit hochenergetischer Teilchenstrahlung bestrahlt wird. Üblicherweise werden hierzu Protonen oder Kohlenstoffionen verwendet, aber auch andere Partikelarten, wie beispielsweise Pionen oder Heliumionen können eingesetzt werden.

Der Vorteil der Partikeltherapie gegenüber der konventionellen Strahlentherapie mit Gammastrahlen ist ein anderes Wechselwirkungsverhalten der Partikel mit dem Gewebe. Solange die Partikel eine hohe Energie (Größenordnung von > 50 MeV/u) besitzen, ist ihre Wechselwirkung mit dem Gewebe nur relativ gering. Erst nachdem sie beim Durchgang durch Gewebe an Energie verloren haben, nimmt die Wechselwirkung zu. Auf einer Strecke in der Größenordnung von wenigen Millimetern findet der Hauptteil der Wechselwirkung mit dem Gewebe statt, um darauf weit gehend auf Null abzufallen. Das dabei erzeugte Tiefendosisprofil bezeichnet man als Bragg-Peak. Aufgrund dieses Verhaltens ist es möglich, die Energie des Teilchenstrahls gezielt beispielsweise auf einen Tumor im Inneren des Körpers zu richten unter gleichzeitiger Schonung des umgebenden Gewebes bzw. von Risikoorganen. Die Eindringtiefe der Teilchen und damit der Ort des Wirkmaximums werden durch die Energie des Teilchenstrahls bestimmt. Bei einer Bestrahlung übliche Energien liegen bei Protonen im Bereich von 48 MeV/u bis 250 MeV/u und im Bereich von Kohlenstoffionen im Bereich von 85 MeV/u bis 430 MeV/u.

Eine Möglichkeit, Partikel auf eine hohe Energie zu beschleunigen, ist die Verwendung eines Zyklotrons. Hierzu werden elektrisch geladene Partikel von einer Ionenquelle erzeugt und anschließend in dem Zyklotron mit starken elektromagnetischen Feldern in einer spiralförmigen Bahn auf eine Zielenergie beschleunigt. Die Partikel werden von der schnellsten Spiralbahn am Rande des Zyklotrons aus dem Zyklotron ausgekoppelt. Nach Verlassen des Zyklotron ist es notwendig, die Energie des Partikelstrahls grob zu justieren, um die Energie des Partikelstrahls an die gewünschte Eindringtiefe anzupassen. Dies erfolgt in einem dem Zyklotron nachgeschalteten, so genannten Energie-Selektions-System. Nach dem Energie-Selektions-System wird der Partikelstrahl über ein Strahltransportsystem zu dem gewünschten Behandlungsplatz geführt. Eine Feinjustierung der Energie des Partikelstrahls kann - falls erforderlich - nach dem Energie-Selektions-System erfolgen.

In letzter Zeit gibt es Bestrebungen, Partikeltherapieanlagen zu bauen, mit denen zwei unterschiedliche Partikelarten beschleunigt und zur Bestrahlung eingesetzt werden können. Im Falle von Zyklotron-basierten Partikeltherapieanlagen geschieht dies beispielsweise dadurch, dass jede Partikelart in einem eigenen, auf die Partikelart abgestimmten Zyklotron beschleunigt wird.

Die EP-A-1 430 932 offenbart eine Partikeltherapieanlage mit zwei Beschleunigereinheiten, die zur Beschleunigung von Partikeln verwendet werden.

Die Schrift von Mizota M et al. "The high-energy beam transport system for HIMAC", Mitsubishi Electric Advance; Mitsubishi Electric Corporation; Tokyo, Seiten 2-4, Bd. 62, 1995, zeigt eine Partikeltherapieanlage mit zwei parallelen Synchrotronringen.

Es ist die Aufgabe der Erfindung, eine Partikeltherapieanlage mit zumindest zwei Beschleunigungseinheiten bereitzustellen, die auf einfache und kostengünstige Weise gebaut und betrieben werden kann.

Die Aufgabe wird erfindungsgemäß durch eine Partikeltherapieanlage nach Anspruch 1 gelöst. Vorteilhafte Ausgestaltungen finden sich in den Merkmalen der abhängigen Ansprüche.

Demnach umfasst die Partikeltherapieanlage zumindest zwei Beschleunigungseinheiten. Dabei können mit jeder der Beschleunigungseinheiten Partikel auf mindestens eine zur Bestrahlung notwendige Energie beschleunigt werden. Den zumindest zwei Beschleunigungseinheiten ist ein gemeinsames Energie-Selektions-System nachgeschaltet, mit dem die Energie von Partikeln, die mit einer der Beschleunigungseinheiten beschleunigt worden sind, reduziert werden kann.

Die Strahlführung nach den Beschleunigungseinheiten ist folglich so gelegt, dass ein gemeinsames Energie-Selektions-System von mehreren Beschleunigungseinheiten, die parallel zueinander angeordnet sind, genutzt werden kann. Die Strahlführung der verschiedenen, aus den Beschleunigungseinheiten austretenden Partikelstrahlen kann also bereits vor dem Energie-Selektions-System zusammengeführt werden. Alternativ dazu ist es beispielsweise möglich, zwei parallele Strahlführungen mit einem einzigen Energie-Selektions-System zu kombinieren, indem beispielsweise das Energie-Selektions-System in derjenigen Strahlführung positioniert wird, in der es gerade benötigt wird.

Da in einem Energie-Selektions-System üblicherweise Partikelstrahlen durch Wechselwirkung mit Materie abgebremst werden, ist die Strahlenbelastung im Bereich eines Energie-Selektions-Systems vergleichsweise hoch. Durch die Verwendung eines gemeinsamen Energie-Selektions-Systems müssen Maßnahmen zur Abschirmung der Strahlenbelastung lediglich einmal ergriffen werden. Hierdurch kann Strahlenschutzanforderungen kostengünstiger entsprochen werden, verglichen mit Partikeltherapieanlagen, bei denen jeder Beschleunigungseinheit ein eigenes Energie-Selektions-System nachgeschaltet ist.

Vorteilhafterweise sind mit den zumindest zwei Beschleunigungseinheiten unterschiedliche Partikelarten beschleunigbar, d.h., dass mit jeder der Beschleunigungseinheiten eine eigene Partikelart beschleunigt werden kann.

Jede Partikelart hat demzufolge ihre eigene Beschleunigungseinheit. Da sich die Partikelarten in den meisten Fällen hinsichtlich ihrer Masse, Ladung und/oder ihres Masse-Ladung-Verhältnisses unterscheiden, können die Beschleunigungseinheiten auf die jeweilige Partikelart abgestimmt werden.

Es schließt sich dem gemeinsamen Energie-Selektions-System ein Strahltransportsystem an, mit denen die Partikel zu einem oder mehreren Behandlungsräumen geführt werden.

Insbesondere, wenn die Beschleunigungseinheiten als Zyklotron-basierte Beschleunigungssysteme ausgebildet sind, ist das gemeinsame nachgeschaltete Energie-Selektions-System vorteilhaft. Der aus einem Zyklotron austretende Partikelstrahl weist eine feste Energie auf. Mit dem vorliegenden Aufbau der Partikeltherapieanlage kann nun die Energie beider Partikelstrahlen mit einem einzigen gemeinsamen Energie-Selektions-System moduliert bzw. reduziert werden. Darüber hinaus kann das Strahltransportsystem, das den Partikelstrahl jeweils von einem Zyklotron-basierten Beschleunigungssystem zu dem Energie-Selektions-System führt, einfacher und kostengünstiger ausgebildet werden, da es nur auf die feste Energie abgestimmt werden muss, mit der der Partikelstrahl aus dem Zyklotron austritt. Der Aufbau mit dem gemeinsamen nachgeschalteten Energie-Selektions-System kann jedoch auch bei anderen Beschleunigungssystemen wie beispielsweise Synchrotronbasierten Beschleunigungssystemen angewendet werden.

Als Partikelarten, die zur Beschleunigung und Bestrahlung eingesetzt werden, können beispielsweise Protonen oder Kohlenstoffionen verwendet werden. Der Aufbau der Partikeltherapieanlage mit einem gemeinsamen Energie-Selektions-System kann insbesondere bei Partikeltherapieanlagen Einsatz finden, die zur gemeinsamen Verwendung von Protonen und Kohlenstoffionen ausgebildet sind.

Das gemeinsame Energie-Selektions-System kann zumindest ein keilförmig oder plattenförmig ausgebildetes Element umfassen, wodurch ein einfacher Aufbau des Energie-Selektion-Systems möglich ist.

Ausführungsformen der vorliegenden Erfindung und vorteilhafte Ausgestaltungen gemäß den Merkmalen der abhängigen Ansprüche werden anhand der nachfolgenden Zeichnung näher erläutert, ohne jedoch darauf beschränkt zu sein. Es zeigen
- Figur 1: einen schematischen Aufbau über eine Zyklotron- basierte Partikeltherapieanlage,
- Figur 2: einen schematischen Aufbau eines Energie-Selektions- Systems mit keilförmigen Elementen, und
- Figur 3: einen schematischen Aufbau eines Energie-Selektions- Systems mit plattenförmigen Elementen.

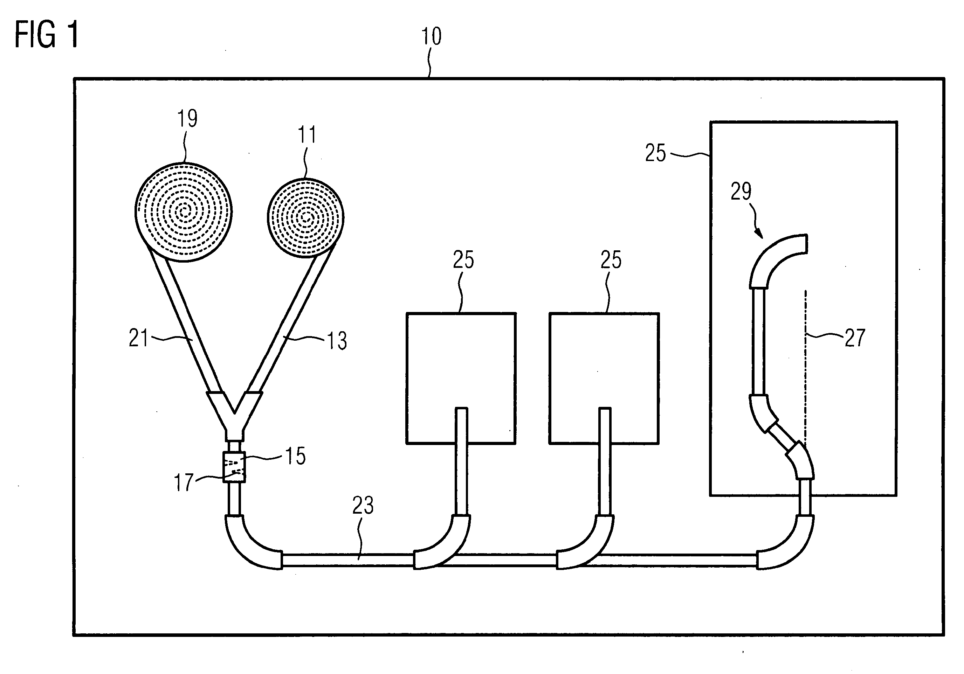

In Figur 1 ist ein schematischer Aufbau einer Partikeltherapieanlage 10 gezeigt, bei der zwei unterschiedliche Partikelarten zur Bestrahlung insbesondere von Tumoren verwendet werden können. Dies können beispielsweise Protonen und Kohlenstoffionen sein.

In einem ersten Zyklotron 11 wird eine erste Partikelart, die von einer nicht dargestellten Quelle erzeugt wird, auf eine erste Zielenergie beschleunigt. Der so entstehende Partikelstrahl wird aus dem ersten Zyklotron 11 ausgekoppelt und über ein dem Zyklotron 11 nachfolgendes erstes Strahltransportsystem 13 zu einem Energie-Selektions-System 15 geführt.

Wenn beispielsweise in dem ersten Zyklotron 11 Protonen auf eine Energie von 230 MeV beschleunigt werden, kann die Energie des Protonenstrahls im Energie-Selektions-System 15 auf eine variabel einstellbare Energie zwischen 230 MeV und 70 MeV abgebremst werden.

In einem zweiten Zyklotron 19 wird eine zweite Partikelart, die ebenfalls von einer nicht dargestellten Quelle erzeugt wird, auf eine zweite Zielenergie beschleunigt. Der so entstehende Partikelstrahl wird aus dem zweiten Zyklotron 19 ausgekoppelt und über ein dem Zyklotron 19 nachfolgendes zweites Strahltransportsystem 21 zu demselben Energie-Selektions-System 15 geführt. In dem Energie-Selektions-System 15 wird die Energie des zweiten Partikelstrahls wie oben für den ersten Partikelstrahl bzw. für Protonen beschrieben auf eine gewünschte Energie eingestellt. Das erste Zyklotron 11 und das zweite Zyklotron 19 können dabei - wie dargestellt - nebeneinander oder aber auch beliebig zueinander, insbesondere vertikal übereinander vertikal übereinander angeordnet werden.

Je nachdem, mit welcher Partikelart die Partikeltherapieanlage betrieben werden soll, wird die Erzeugung des Partikelstrahls mit dem ersten oder mit dem zweiten Zyklotron betrieben.

Die zwischen den Zyklotronen und dem Energie-Selektions-System eingesetzten Strahltransportsysteme 13, 21 können dabei günstiger ausgebildet werden, da sie jeweils nur auf einen Partikelstrahl der ersten Partikelart mit der ersten Zielenergie bzw. auf einen Partikelstrahl der zweiten Partikelart mit der zweiten Zielenergie abgestimmt werden müssen. Beispielsweise können in diesem Abschnitt des Strahltransportsystems eingesetzte Magnete einfacher ausgebildet werden.

Nachdem der Partikelstrahl das Energie-Selektions-System 15 verlassen hat, wird der Partikelstrahl über das nachfolgende Strahltransportsystem 23 zu den einzelnen Bestrahlungs- bzw. Behandlungsräumen 25 geführt. In der hier gezeigten Figur sind drei Bestrahlungsräume 25 dargestellt. In einem Bestrahlungsraum 25 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 27 bewegliche rotierbare Gantry 29 von verschiedenen Richtungen aus.

Da der Partikelstrahl geladen ist, kann er mit einem geeignet ausgebildeten Magnetsystem quer zur Strahlrichtung abgelenkt werden. Bei einem speziellen Bestrahlungsverfahren, dem so genannten Rasterscan-Verfahren, wird der Partikelstrahl mit einer Fokusgröße von wenigen Millimetern schichtweise über das Zielvolumen gescannt und erlaubt damit eine genaue, Tumor-konforme Bestrahlung. Zur schichtweisen Bestrahlung wird die Energie des Partikelstrahls entsprechend fein abgestimmt. Es sind aber auch andere Bestrahlungsverfahren möglich, wie beispielsweise ein Spotscan-Verfahren.

Es können aber auch andere Bestrahlungsverfahren mit so genannten passiven Strahlformungselementen eingesetzt werden. Hierbei wird der Partikelstrahl beispielsweise aufgeweitet und anschließend mithilfe eines Kollimatoren und/oder mithilfe von strahlformenden Elementen, die in den Strahlengang eingebracht werden, an die Form eines Tumors angepasst.

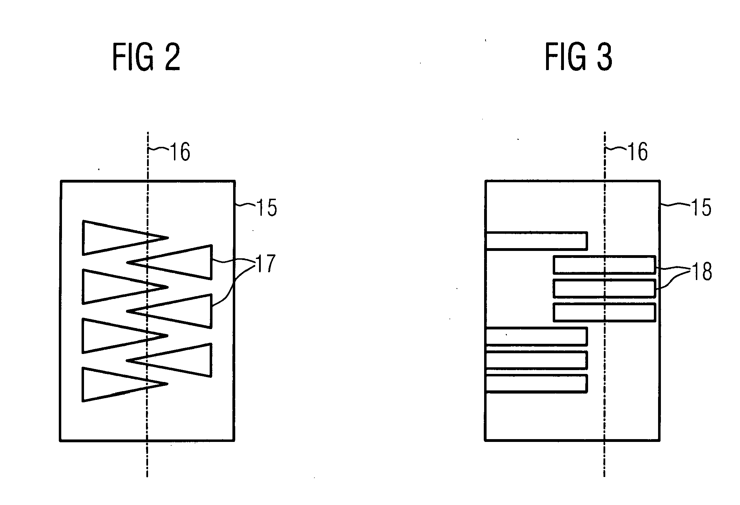

Figur 2 und Figur 3 zeigen schematisch verschiedene Ausführungsformen eines Energie-Selektions-Systems 15. Bei dem in Figur 2 gezeigten Energie-Selektions-System 15 kann die Energie des Partikelstrahls, der aufgrund der Beschleunigung durch ein Zyklotron eine feste Energie aufweist, auf eine gewünschte Größe reduziert werden, indem keilförmige Elemente 17, beispielsweise aus Kohlenstoff, in den Strahlengang 16 eingebracht werden. Je stärker die keilförmigen Elemente 17 in den Strahlengang 16 eingebracht werden, desto stärker wird die Energie des Partikelstrahls reduziert.

In ähnlicher Weise funktioniert das Energie-Selektions-System 15, das in Figur 3 dargestellt ist. Hier können plattenförmig ausgebildete Elemente 18 variabel in den Strahlengang 16 eingebracht werden. Je nach Gesamtdicke der plattenförmigen Elemente 18, durch die der Partikelstrahl hindurch geht, wird seine Energie entsprechend reduziert.

## Patentansprüche

1. Partikeltherapieanlage, umfassend:
- zumindest zwei parallel zueinander angeordnete Beschleunigungseinheiten (11, 19), wobei mit jeder der Beschleunigungseinheiten (11, 19) Partikel auf mindestens eine zur Bestrahlung notwendige Energie beschleunigbar sind,
**dadurch gekennzeichnet, dass**
die Partikeltherapieanlage weiterhin umfasst:
- ein gemeinsames, den zumindest zwei parallel zueinander angeordneten Beschleunigungseinheiten (11, 19) nachgeschaltetes Energie-Selektions-System (15), mit dem die Energie von Partikeln, die mit einer der Beschleunigungseinheiten (11, 19) beschleunigt worden sind, reduzierbar ist,
wobei sich dem gemeinsamen Energie-Selektions-System (15) ein Strahltransportsystem (23) strahlabwärts anschließt, mit dem die Partikel zu zumindest einem Behandlungsraum geführt werden können.

2. Partikeltherapieanlage nach Anspruch 1, wobei mit den zumindest zwei Beschleunigungseinheiten (11, 19) unterschiedliche Partikelarten beschleunigbar sind.

3. Partikeltherapieanlage nach Anspruch 2, wobei eine der Partikelarten Protonen oder Kohlenstoffionen sind.

4. Partikeltherapieanlage nach einem der Ansprüche 1 bis 3, wobei zumindest eine der Beschleunigungseinheiten (11, 19) ein Zyklotron-basiertes Beschleunigungssystem ist.

5. Partikeltherapieanlage nach einem der Ansprüche 1 bis 4, wobei das gemeinsame Energie-Selektions-System (15) zumindest ein keilförmig oder plattenförmig ausgebildetes Element umfasst.

## Claims

1. Particle therapy system, comprising:
- at least two acceleration units (11, 19) disposed parallel to one another, each of the acceleration units (11, 19) being able to accelerate particles to at least one energy required for irradiation,
**characterised in that**
the particle therapy unit further comprises:
- a common energy selection system (15) connected downstream of the at least two acceleration units (11, 19) disposed parallel to one another, the common energy selection system (15) being able to reduce the energy of particles that have been accelerated using one of the acceleration units (11, 19), wherein a beam transporting system (23) that is able to carry the particles to at least one treatment room is connected downstream of the common energy selection system (15).

2. Particle therapy system according to claim 1, wherein the at least two acceleration units (11, 19) are able to accelerate different types of particles.

3. Particle therapy system according to claim 2, wherein one of the types of particles are protons or carbon ions.

4. Particle therapy system according to one of claims 1 to 3, wherein at least one of the acceleration units (11, 19) is a cyclotron-based acceleration system.

5. Particle therapy system according to one of claims 1 to 4, wherein the common energy selection system (15) comprises at least one element that is configured as wedge-shaped or plate-shaped.

## Revendications

1. Installation de thérapie par particules, comprenant .
- au moins deux unités d'accélération (11, 19) disposées parallèlement l'une à l'autre, chacune desdites unités d'accélération (11, 19) permettant d'accélérer des particules à au moins une énergie nécessaire à l'irradiation,
**caractérisée en ce que**
l'installation de thérapie par particules comprend en outre :
- un système commun de sélection d'énergie (15) monté en aval desdites au moins deux unités d'accélération (11, 19) disposées parallèlement l'une à l'autre, permettant de réduire l'énergie de particules ayant été accélérées par l'une des unités d'accélération (11, 19),
ledit système commun de sélection d'énergie (15) étant suivi, en aval, d'un système de transport de faisceau (23) permettant de conduire les particules vers au moins une salle de traitement.

2. Installation de thérapie par particules selon la revendication 1, lesdites au moins deux unités d'accélération (11, 19) permettant d'accélérer différents types de particules.

3. Installation de thérapie par particules selon la revendication 2, l'un des types de particules étant des protons ou des ions carbone.

4. Installation de thérapie par particules selon l'une des revendications 1 à 3, au moins l'une desdites unités d'accélération (11, 19) étant un système d'accélération (11, 19) sur la base d'un cyclotron.

5. Installation de thérapie par particules selon l'une des revendications 1 à 4, ledit système commun de sélection d'énergie (15) comprenant au moins un élément réalisé en forme de coin ou en forme de plaque.
